# FASCICULE DE BREVET EUROPEEN

(11) **EP 1 744 690 B1**
(45) Date de publication et mention de la délivrance du brevet: **14.09.2011**
(21) Numéro de dépôt: 05769961.3
(22) Date de dépôt: 09.05.2005
(51) Int. Cl.: A61B 17/70

(54) **IMPLANT INTERVERTEBRAL**
BANDSCHEIBENIMPLANTAT
INTERVERTEBRAL IMPLANT

(30) Priorité: 11.05.2004 FR 0405064
(43) Date de publication de la demande: 24.01.2007
(73) Titulaire: ZIMMER SPINE, 33000 Bordeaux (FR)
(72) Inventeur: LE COUEDIC, Régis, F-78570 ANDRESY (FR); PASQUET, Denis, F-33360 Quinsac (FR)
(74) Mandataire: Intès, Didier Gérard André
(86) Numéro de dépôt international: PCT/FR2005/001140
(87) Numéro de publication internationale: WO 2005/120369

(56) Documents cités:
- WO-A-03/015645
- FR-A- 2 799 640
- FR-A- 2 822 051
- US-A- 5 496 318
- US-A- 6 156 038

## Description

La présente invention a pour objet un implant intervertébral pour la partie courante de la colonne vertébrale, et plus particulièrement la cale de l'implant.

Les cales intervertébrales sont des dispositifs bien connus qui sont placés entre deux vertèbres adjacentes pour solidariser ces deux vertèbres et maintenir entre celles-ci un intervalle fixe. La demande de brevet français FR 2 822 051 A1 au nom du demandeur décrit de telles cales (Le préambule de la revendiction 1 est basé sur ce document). Il suffit de rappeler que la cale est munie à chacune de ses extrémités d'une échancrure dans laquelle vient s'engager l'apophyse épineuse de la vertèbre. Un système de liens ou de tresses permet de solidariser chaque extrémité de la cale avec l'épineuse.

Cependant, la mise en place d'une telle cale soulève certains problèmes liés à la pratique chirurgicale. En effet, un ligament, dit supra épineux, relie tous les sommets des apophyses épineuses entre eux. Pour mettre en place la cale, il faut déplacer ce ligament. Pratiquement, on le détache des deux apophyses concernées et on l'écarte au moyen d'un instrument chirurgical approprié. Pour détacher le ligament des épineuses, on utilise un scalpel. Une fois que la cale est mise en place, on recoud le ligament aux épineuses après avoir pratiqué une petite ouverture dans ces dernières pour placer le fil de couture.

L'inconvénient majeur de cette pratique chirurgicale est qu'en touchant au ligament pour le détacher puis l'écarter, on lui fait perdre ses propriétés mécaniques. De plus, toutes ces opérations nécessitent du temps qui augmente la durée de l'intervention chirurgicale.

Pour remédier à cet inconvénient, on a déjà proposé des cales intervertébrales constituées par deux parties distinctes. Chaque partie de la cale est mise en place en contournant le ligament supra épineux puis à l'aide d'instruments chirurgicaux adéquats, on solidarise les deux parties de la cale. De telles cales en deux parties sont notamment décrites dans le brevet US 6 156 038, ou dans la demande PCT publiée WO 03/015645 A1.

Cependant, les cales en deux parties décrites dans ce document sont d'une utilisation relativement délicate. En particulier, l'assemblage des deux parties de la cale in situ est relativement délicat et il n'est pas sûr d'obtenir l'assemblage des deux pièces formant la cale de façon convenable.

Un objet de la présente invention est de fournir une cale intervertébrale constituée par deux pièces distinctes dont l'assemblage soit plus aisé pour le chirurgien lors de l'intervention.

Pour atteindre ce but, selon l'invention, on fournit un implant intervertébral selon la revendication 1 annexée. Un tel implant intervertébral comprend une cale constituée par une première et une deuxième pièce, lesdites pièces étant munies de moyens d'assemblage mutuel. Ces moyens d'assemblage comprennent :
- des premiers moyens de clipsage solidaires d'une desdites pièces,
- des deuxièmes moyens de clipsage solidaires de l'autre pièce, aptes à coopérer avec les premiers moyens de clipsage pour réaliser un clipsage désenclipsable des deux pièces selon une direction de clipsage, lesdits premiers et deuxièmes moyens de clipsage faisant partie intégrante desdites deux pièces ; et
- des moyens de guidage des deux pièces lors de leur enclipsage, lesdits moyens de guidage étant distincts des moyens de clipsage et comprenant :
   .. un premier ensemble de guidage solidaire d'une desdites pièces et sensiblement non déformable ; et
   .. un deuxième ensemble de guidage solidaire de l'autre pièce et sensiblement non déformable, pour coopérer avec le premier ensemble de guidage afin de réaliser un guidage mutuel des deux pièces selon ladite direction de clipsage et un positionnement relatif des deux pièces dans un plan orthogonal à la direction de clipsage.

On comprend que, grâce au fait que chaque pièce constituant la cale intervertébrale comporte, d'une part, des moyens de guidage et d'autre part, des moyens de clipsage, les moyens de guidage assurent le positionnement relatif des moyens de clipsage de façon rigoureuse et de manière aisée pour le chirurgien. On assure ainsi le bon assemblage des deux pièces constituant la cale et de plus, cette opération peut être réalisée plus rapidement qu'avec les cales de l'état de la technique.

En outre, comme les moyens de clipsage font partie intégrante des deux pièces, la mise en place de l'implant par le chirurgien est facilitée.

Selon un mode préféré de réalisation de l'invention, chaque pièce de la cale comporte une face d'assemblage destinée à être appliquée contre celle de l'autre pièce lorsque les deux pièces sont assemblées.

De préférence encore, les moyens de guidage sont constitués respectivement par deux pions de guidage faisant saillie hors d'une des faces d'assemblage et par deux évidements débouchant dans la face d'assemblage de l'autre pièce. On obtient ainsi un guidage efficace d'une pièce par rapport à l'autre en donnant aux pions de guidage une forme convenable.

D'autres caractéristiques et avantages de l'invention apparaîtront mieux à la lecture de la description qui suit d'un mode de réalisation de l'invention donné à titre d'exemple non limitatif. La description se réfère aux figures annexées sur lesquelles :
- la figure 1 est une vue éclatée des différents éléments constituant l'implant intervertébral ;
- la figure 2 est une vue de la face d'assemblage d'une des deux pièces constituant la cale intervertébrale ;
- la figure 3 est une vue de côté de la cale après l'assemblage des deux pièces la constituant ; et
- la figure 4 est une coupe longitudinale de l'élément d'auto-blocage servant à solidariser les tresses de l'implant.

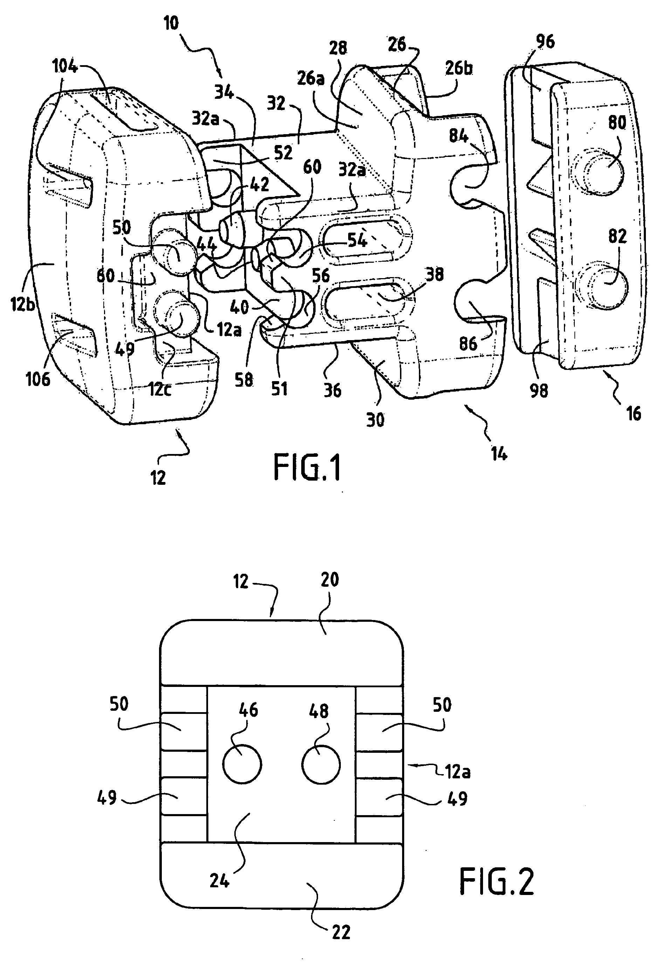

Sur la figure 1, on a représenté les deux pièces 12 et 14 dont l'assemblage permet d'obtenir la cale intervertébrale 10. Sur cette figure, on a également représenté un élément amovible 16 servant à la fixation et au serrage des tresses assurant la solidarisation des deux apophyses épineuses sur la cale. Cet élément de fixation 16 sera décrit ultérieurement. La pièce 12 constitue un premier flanc de la cale intervertébrale. Cette pièce 12 comporte une face interne 12a et une face externe 12b. La face interne 12a définit une surface d'appui supérieure 20, une surface d'appui inférieure 22 et une face d'assemblage médiane 24.

La deuxième pièce 14 constitue également un flanc 26 présentant une face interne 26a tournée vers la face interne 12a de la pièce 12 et une face externe 26b. La face interne 26a du flanc 26 définit une surface supérieure d'appui 28 et une surface inférieure d'appui 30. Dans la région médiane de la face interne du flanc 26, la pièce 14 comporte une projection 32 qui fait saillie dans la partie centrale de la face 26a. Cette projection 32 ou pièce d'espacement définit deux faces d'appui respectivement supérieure 34 et inférieure 36, ces deux faces étant sensiblement parallèles. La pièce d'écartement 32 peut comporter des évidements transversaux tels que 38 pour conférer à cette partie de la cale une certaine élasticité. L'extrémité libre de la pièce d'écartement 32 définit une face d'assemblage 40 destinée à venir en appui sur la face d'assemblage 24 de la pièce 12 lorsque les deux pièces constituant la cale sont assemblées.

Dans les faces d'assemblage 40 et 24 des deux pièces, sont prévus des moyens de guidage mutuel lors du rapprochement de ces deux pièces en vue de leur solidarisation. De préférence, ces moyens de guidage sont constitués par deux pions de guidage 42 et 44 faisant saillie hors de la face d'assemblage 40 de la pièce 14 et par deux évidements borgnes 46 et 48 ménagés dans la face d'assemblage 24 de la pièce 12. On ne sortirait pas de l'invention si on prévoyait un seul pion et un seul évidement. De préférence, comme cela est en soi connu, les extrémités des pions d'assemblage 42 et 44 sont coniques pour assurer le positionnement initial des deux pièces. La solidarisation des pièces 12 et 14 est obtenue par des moyens de clipsage qui sont réalisés respectivement dans les faces latérales 12c et 12d de la pièce 12, et dans les faces latérales 32a et 32b de la projection 32 de la pièce 14. Ces moyens de clipsage consistent, par exemple pour la pièce 12, en deux paires de tétons cylindriques 49 et 50 et dans deux flancs faisant saillie au-delà de la face d'assemblage 44 de la pièce 14, respectivement référencés 51 et 52. Chaque flanc est muni de deux ouvertures 54 et 56 aptes à recevoir les tétons 49 et 50 lors de l'enclipsage. Chaque orifice 54, 56 comporte un becquet déformable 58 et 60. Comme le montre également la figure 1, les tétons 49 et 50 font saillie dans un évidement 60 réalisé dans les faces latérales 12c, 12d de la pièce 12. Il faut ajouter que bien entendu, les pions de guidage 42 et 44 ont une longueur qui est supérieure à celle des flancs 51 et 52 définissant les éléments femelles de clipsage. Quelque soit le mode particulier de réalisation des moyens de clipsage, ceux-ci font partie intégrante respectivement des deux pièces 12 et 14.

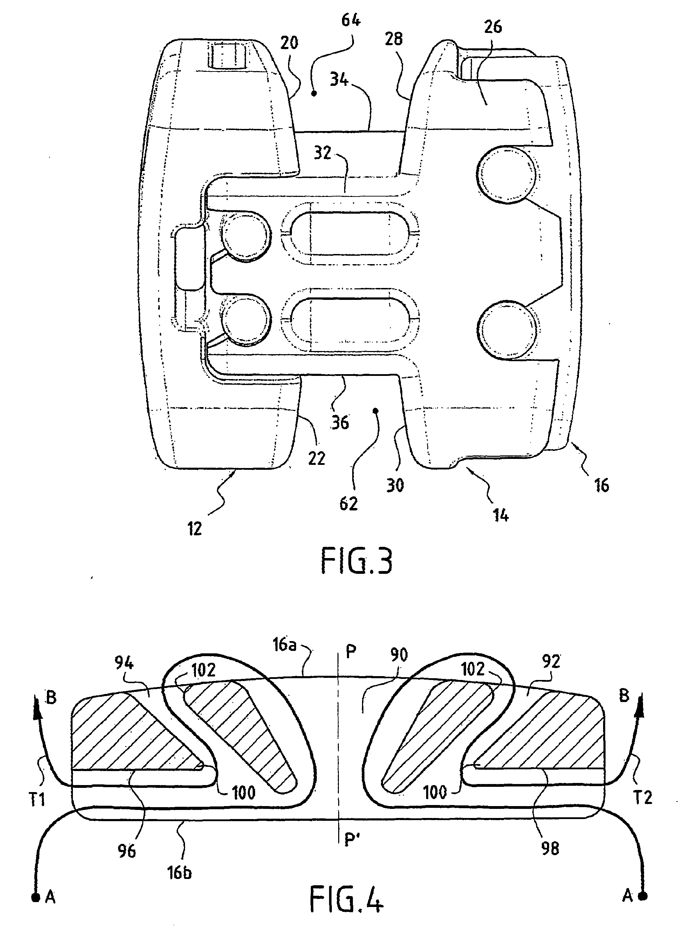

Comme le montre mieux la figure 3, lorsque les pièces 12 et 14 sont assemblées, de la manière qu'on l'expliquera ultérieurement, on obtient une cale intervertébrale ayant la structure habituelle. En particulier, les faces d'appui 20, 22 de la pièce 12 ainsi que les faces d'appui 28, 30 du flanc 26 de la pièce 14 et enfin les faces supérieure 34 et inférieure 36 de l'extension 32 définissent deux évidements 62 et 64 destinés à recevoir les apophyses épineuses des deux vertèbres entre lesquels la cale est disposée.

Il faut ajouter que bien entendu, les pions de guidage 42 et 44 ont une longueur qui est supérieure à celle des flancs 50 et 52 définissant les éléments femelles de clipsage.

Le mode d'utilisation de la cale décrite précédemment est le suivant. Le chirurgien met en place, en contournant le ligament supra épineux, les pièces 12 et 14 constituant la cale. A l'aide d'instruments chirurgicaux adaptés, il rapproche les deux pièces pour faire coopérer les pions de guidage 42 et 44 avec les évidements de guidage 46 et 48. Lorsque le positionnement relatif qui en résulte est réalisé, les organes de clipsage 48, 50 et 54, 56 sont en regard les uns des autres. Il suffit pour le chirurgien d'exercer une pression sur les faces externes des deux pièces pour provoquer le clipsage des pièces 12 et 14, et donc l'obtention de la cale complète 10. Il faut ajouter que les éléments de clipsage 48, 50 et 54, 56 sont réalisés de telle manière qu'ils assurent un clipsage efficace mais qu'il soit possible de les désenclipser dans le cas où il serait nécessaire de procéder au changement de la cale. Comme on le voit, la mise en place de la cale est aisée puisque celle-ci ne comprend que deux pièces.

En se référant maintenant aux figures 1 et 4, on va décrire un mode préféré de réalisation des moyens de solidarisation de la cale 10 et des apophyses épineuses des vertèbres. Ce système de solidarisation est constitué par la pièce de blocage ou de solidarisation 16 et par deux tresses T1 et T2. La pièce de solidarisation 16 peut être fixée sur la face externe 26b de la pièce 14 par des moyens de clipsage 80, 82, 84 et 86 qui sont identiques aux moyens de clipsage des pièces 12 et 14.

La pièce de fixation 16 présente une face de clipsage 16b sur la pièce 14 et une face externe 16a. La pièce de fixation 16 présente un plan de symétrie P, P' orthogonal aux faces 16a et 16b et parallèle aux faces 32 et 34 de l'extension 30 de la pièce 14. La pièce de blocage 16 comporte une fente centrale 90 qui débouche dans les deux faces de la pièce 16 et deux fentes latérales inclinées symétriques 92 et 94 qui débouchent également dans les deux faces de la pièce 16. La face de clipsage 16b de la pièce 16 présente deux surfaces en retrait 96 et 98 qui s'étendent entre respectivement les fentes latérales 92 et 94 et les extrémités supérieure et inférieure de la pièce 16 qui constituent des surfaces de serrage des tresses. La fente 92 et la surface en retrait 98 définissent une première arête 100. De même, la fente 92 avec la face externe 16a de la pièce 16 définissent une deuxième arête aiguë 102 parallèle à l'arête 100. Comme le montre la figure, l'arête 102 est plus éloignée du plan médian P, P' que l'arête 100. En outre, il faut préciser que les surfaces de serrage 96 et 98 sont disposées de telle manière que lorsque la pièce 16 est clipsée sur la cale, la distance entre la face 26b de la cale et la surface 96 ou 98 soit légèrement inférieure à deux fois l'épaisseur des tresses T1 et T2.

Chaque tresse T1 et T2 comprend une première extrémité A qui est solidaire de la pièce 12. Pour cela, dans l'exemple considéré, la pièce 12 est munie d'une fente supérieure 104 et d'une fente inférieure 106 dans lesquelles les extrémités A des tresses peuvent former une boucle. Après couture de cette boucle, on obtient effectivement la solidarisation des tresses T1 et T2 à chaque extrémité de la pièce 12. La tresse T2 est engagée entre la surface 98 et la face 26b de la cale puis dans la fente centrale 90. Ensuite, elle passe sur une portion de la face externe 16a de la pièce 16 et pénètre dans la fente 92 en passant en regard de l'arête 102. Ensuite, la tresse T2 est engagée en dessous de la surface de pression 98, entre le premier brin de cette même tresse et cette surface. Lorsque le chirurgien exerce une traction sur l'extrémité B de la tresse T2, cela permet d'assurer le serrage de la tresse T2 sur l'apophyse épineuse de la vertèbre supérieure ou inférieure. La tresse T1 est bien sûr engagée dans la pièce 16 de la même manière pour assurer la liaison avec l'apophyse épineuse de l'autre vertèbre.

On comprend que la pièce de solidarisation 16 forme pour chaque tresse un système autobloquant. Lorsque le chirurgien exerce une traction sur l'extrémité libre B de la tresse, celle-ci peut se déplacer sans frottement excessif dans les fentes 90 et 92 jusqu'à ce que l'on obtienne l'effet de serrage souhaité. En revanche, lorsqu'aucune traction n'est exercée sur l'extrémité B de la tresse, c'est-à-dire en utilisation normale de l'implant intervertébral, un effet d'autoblocage est obtenu grâce à la présence des arêtes 100 et 102 et à l'effet de serrage de la surface de pression 98 sur les deux brins de la tresse.

Selon un mode préféré de réalisation, les premiers moyens de clipsage comprennent au moins deux organes mâles de clipsage 49 et 50 sensiblement non déformables, disposés de part et d'autre de la surface d'assemblage 24 et les deuxièmes moyens de clipsage comprennent au moins deux organes femelles 54 et 56 de clipsage élastiquement déformables disposés de part et d'autre de la surface d'assemblage 40.

De préférence également, ledit premier ensemble de guidage comprend au moins un pion de guidage 42 et 40 faisant saillie dans une face d'assemblage 40 d'une des pièces et au moins un évidement 46 et 48 débouchant dans la face d'assemblage 24 de l'autre pièce.

## Revendications

1. Implant intervertébral comprenant une cale (10) constituée par une première (12) et une deuxième (14) pièce, chacune des deux pièces (12, 14) ayant une face d'assemblage (24, 40), lesdites faces d'assemblage étant appliquées l'une contre l'autre lorsque les deux pièces sont assemblées, lesdites pièces (12, 14) étant munies de moyens d'assemblage mutuel,
lesdits moyens d'assemblage mutuel comprennant :
- des premiers moyens de clipsage (54, 56) solidaires d'une desdites pièces,
- des deuxièmes moyens de clipsage (49, 50) solidaires de l'autre pièce, aptes à coopérer avec les premiers moyens de clipsage pour réaliser un clipsage désenclipsable des deux pièces (12, 14) selon une direction de clipsage, lesdits premiers et deuxièmes moyens de clipsage (49, 50; 54, 56) faisant partie intégrante des deux pièces (12, 14); cet implant étant **caractérisé en ce que** les moyens d'assemblage mutuel comprennent
des moyens de guidage des deux pièces (12, 14) lors de leur enclipsage, lesdits moyens de guidage étant distincts des premiers et deuxièmes moyens de clipsage et comprenant :
- un premier ensemble de guidage (42, 44) solidaire d'une desdites pièces, situé sur la face d'assemblage (40) de cette pièce, et sensiblement non déformable; et
- un deuxième ensemble de guidage (46, 48) solidaire de l'autre pièce, situé sur la face d'assemblage (24) de cette autre pièce, et sensiblement non déformable, pour coopérer avec le premier ensemble de guidage afin de réaliser un guidage mutuel des deux pièces (12, 14) selon ladite direction de clipsage et un positionnement relatif des deux pièces (12, 14) dans un plan orthogonal à la direction de clipsage.

2. Implant intervertébral selon la revendication 1, dans lequel les premiers moyens de clipsage comprennent au moins deux organes mâles de clipsage (49, 50) sensiblement non déformables, disposés de part et d'autre de la surface d'assemblage et les deuxièmes moyens de clipsage comprennent au moins deux organes femelles (54, 56) de clipsage élastiquement déformables disposés de part et d'autre de la surface d'assemblage.

3. Implant intervertébral selon la revendication 1 ou 2, dans lequel ledit premier ensemble de guidage comprend au moins un pion de guidage (42, 44) faisant saillie sur une face d'assemblage (40) d'une des pièces (12, 14) et le deuxième ensemble de guidage comprend au moins un évidement (46, 48) débouchant dans la face d'assemblage (24) de l'autre pièce.

4. Implant intervertébral selon la revendication 3, dans lequel ledit premier ensemble de guidage comprend deux pions de guidage (42, 44) et le deuxième ensemble de guidage comprend deux évidements (46, 48).

5. Implant intervertébral selon l'une quelconque des revendications 1 à 4, dans lequel la première pièce (12) constitue un premier flanc de la cale (10), cette première pièce présentant une face externe (12b) et une face interne (12a) qui définit une surface d'appui supérieure (20), une surface d'appui inférieure (22) et ladite face d'assemblage (24) en position médiane, et dans lequel la deuxième pièce (14) constitue un deuxième flanc de la cale (10), cette deuxième pièce présentant une face externe (26b) et une face interne (26a) qui est tournée vers la face interne (12a) de la première pièce (12) et qui définit une surface d'appui supérieure (28), une surface d'appui inférieure (30) et ladite face d'assemblage (40) en position médiane.

6. Implant selon l'une quelconque des revendications 1 à 5, dans lequel l'une des deux pièces (14) comporte une face externe (26b) opposée à sa face d'assemblage (40), l'implant comprenant, en outre :
- deux tresses (T₁, T₂) de fixation de ladite cale (10) sur deux apophyses épineuses, et
- un élément d'autoblocage (16) comportant des moyens de fixation sur ladite face externe (26b),
chaque tresse (T₁, T₂) ayant une première extrémité solidaire de l'autre pièce (12, 14), non associée à l'élément d'autoblocage (16), et une deuxième extrémité engageable dans ledit élément d'autoblocage (16).

7. Implant selon la revendication 6, dans lequel ledit élément d'autoblocage (16) est fixé sur ladite face externe (26b) de manière amovible.

8. Implant selon l'une quelconque des revendications 1 à 7, dans lequel les extrémités des pions de guidage (42, 44) sont coniques.

## Claims

1. An intervertebral implant comprising a spacer (10) constituted by a first (12) and a second part (14), each of said two parts (12, 14) having an assembly face (24, 40), said assembly faces being pressed one against the other when the parts are assembled together, said parts (12, 14) being provided with mutual assembly means, said mutual assembly means comprising:
first clip-fastener means (54, 56) secured to one of said parts;
second clip-fastener means (49, 50) secured to the other part, suitable for co-operating with the first clip-fastener means to achieve releasable clip-fastening between the two parts (12, 14) in a clip-fastening direction, said first and second clip-fastening means (49, 50; 54, 56) forming integral portions of the two parts (12, 14);
this implant being **characterized in that** the mutual assembly means comprises guide means for guiding the two parts (12, 14) during clip-fastening, said guide means being distinct from the first and second clip-fastener means and comprising:
a substantially non-deformable first guide assembly (42, 44) secured to one of said parts and located on the assembly face (40) of this part; and
a substantially non-deformable second guide assembly (46, 48) secured to the other part and located on the assembly face (24) of this other part, to cooperate with the first guide assembly so as to provide mutual guidance of the two parts (12, 24) along said clip-fastening direction and provide relative positioning of the two parts (12, 24) in a plane orthogonal to the clip-fastening direction.

2. An intervertebral implant according to claim 1, wherein the first clip-fastener means comprise at least two substantially non-deformable male clip-fastener members (49, 50) disposed on either side of the assembly surface, and the second clip-fastener means comprise at least two elastically deformable female clip-fastener members (54, 56) disposed on either side of the assembly surface.

3. An intervertebral implant according to claim 1 or 2, wherein said first guide assembly comprises at least one guide stud (42, 44) projecting from an assembly face (40) of one of the parts (12, 14), and the second guide assembly comprises at least one recess (46, 48) opening out into the assembly face (24) of the other part.

4. An intervertebral implant according to claim 3, wherein said first guide assembly comprises two guide studs (42, 44) and the second guide assembly comprises two recesses (46, 48).

5. An intervertebral implant according to any one of claims 1 to 4, wherein the first part (12) constitutes a first side piece of the spacer (10), the first part (12) having an inside face (12a) and an outside face (12b), the inside face (12a) defining a top bearing surface (20), a bottom bearing surface (22) and said assembly face (24) in a median position, and wherein the second part (14) constitutes a second side piece of the spacer (10), the second part (14) having an outside face (26b) and an inside face (26a), the inside face (26a) facing towards the inside face (12a) of the first part (12) and defining a top bearing surface (28), a bottom bearing surface (30) and said assembly face (40) in a median position.

6. An implant according to any one of claims 1 to 5, wherein one of said two parts (14) has an outside face (26b) opposite to its assembly face (40), the implant further comprising:
two braids (T1, T2) for fastening said spacer (10) on two spinous processes; and
a self-locking element (16) comprising fastener means on said outside face (26b);
each braid (T1, T2) having a first end secured to the other part (12, 14) not associated with the self-locking element (16), and a second end that is engageable in said self-locking element (16).

7. An implant according to claim 6, wherein said self-locking element (16) is removably secured to said outside face (26b).

8. An implant according to any one of claims 1 to 7, wherein the ends of said guide studs (42, 44) are conical.

## Patentansprüche

1. Zwischenwirbelimplantat umfassend einen Keil (10), der von einem ersten (12) und einem zweiten (14) Teil gebildet ist, wobei ein jedes der beiden Teile (12, 14) eine Verbindungsfläche (24, 40) aufweist, wobei die Verbindungsflächen aneinander liegen, wenn die beiden Teile zusammengefügt sind, wobei die Teile (12, 14) mit Mitteln zum gegenseitigen Verbinden ausgestattet sind, wobei die Mittel zum gegenseitigen Verbinden umfassen:
- erste Festclipsmittel (54, 56), die mit einem der Teile fest verbunden sind,
- zweite Festclipsmittel (49, 50), die mit dem anderen Teil fest verbunden sind, welche geeignet sind, mit den ersten Festclipsmitteln zusammenzuwirken, um ein nicht wieder lösbares Festclipsen der beiden Teile (12, 14) in einer Einclipsrichtung zu bewirken, wobei die ersten und die zweiten Festclipsmittel (49, 50; 54, 56) einen Bestandteil der beiden Teile (12, 14) bilden,
wobei dieses Implantat **dadurch gekennzeichnet ist, daß** die Mittel zum gegenseitigen Verbinden Mittel zum Führen der beiden Teile (12, 14) bei ihrem Festclipsen umfassen, wobei die Führungsmittel von den ersten und zweiten Festclipsmitteln getrennt sind und umfassen:
- eine erste Führungsanordnung (42, 44), die mit einem der Teile fest verbunden ist, an der Verbindungsfläche (40) dieses Teils gelegen und im wesentlichen nicht verformbar ist, und
- eine zweite Führungsanordnung (46, 48), die mit dem anderen Teil fest verbunden ist, an der Verbindungsfläche (24) dieses anderen Teils gelegen und im wesentlichen nicht verformbar ist, um mit der ersten Führungsanordnung zusammenzuwirken, um so ein gegenseitiges Führen der beiden Teile (12, 14) in der Einclipsrichtung sowie ein Relativpositionieren der beiden Teile (12, 14) in einer zu der Einclipsrichtung orthogonalen Ebene zu bewirken.

2. Zwischenwirbelimplantat nach Anspruch 1, wobei die ersten Festclipsmittel wenigstens zwei im wesentlichen nicht verformbare einzusteckende Festclipsorgane (49, 50), die auf beiden Seiten der Verbindungsfläche angeordnet sind, umfassen und die zweiten Festclipsmittel wenigstens zwei elastisch verformbare aufnehmende Festclipsorgane (54, 56), die auf beiden Seiten der Verbindungsfläche angeordnet sind, umfassen.

3. Zwischenwirbelimplantat nach Anspruch 1 oder 2, wobei die erste Führungsanordnung wenigstens einen Führungsstift (42, 44), der an einer Verbindungsfläche (40) von einem der Teile (12, 14) vorspringt, umfaßt und die zweite Führungsanordnung wenigstens eine Ausnehmung (46, 48), welche in die Verbindungsfläche (24) des anderen Teils mündet, umfaßt.

4. Zwischenwirbelimplantat nach Anspruch 3, wobei die erste Führungsanordnung zwei Führungsstifte (42, 44) und die zweite Führungsanordnung zwei Ausnehmungen (46, 48) umfaßt.

5. Zwischenwirbelimplantat nach einem der Ansprüche 1 bis 4, wobei das erste Teil (12) eine erste Flanke des Keils (10) bildet, wobei dieses erste Teil eine Außenseite (12b) und eine Innenseite (12a) aufweist, welche eine obere Anlagefläche (20), eine untere Anlagefläche (22) und die Verbindungsfläche (24) in mittlerer Position definiert, und wobei das zweite Teil (14) eine zweite Flanke des Keils (10) bildet, wobei dieses zweite Teil eine Außenseite (26b) und eine Innenseite (26a) aufweist, welche der Innenseite (12a) des ersten Teils (12) zugewandt ist und eine obere Anlagefläche (28), eine untere Anlagefläche (30) sowie die Verbindungsfläche (40) in mittlerer Position definiert.

6. Implantat nach einem der Ansprüche 1 bis 5, wobei das eine der beiden Teile (14) eine seiner Verbindungsfläche (40) gegenüberliegende Außenseite (26b) aufweist, wobei das Implantat ferner umfaßt:
- zwei Schnüre (T₁, T₂) zum Befestigen des Keils (10) an zwei Dornfortsätzen und
- ein Selbstsicherungselement (16), das Mittel zum Befestigen an der Außenseite (26b) umfaßt,
wobei jede Schnur (T1, T₂) ein erstes Ende, das mit dem anderen, nicht dem Selbstsicherungselement (16) zugeordneten Teil (12, 14) fest verbunden ist, sowie ein zweites Ende, das in das Selbstsicherungselement (16) einsteckbar ist, aufweist.

7. Implantat nach Anspruch 6, wobei das Selbstsicherungselement (16) an der Außenseite (26b) lösbar befestigt ist.

8. Implantat nach einem der Ansprüche 1 bis 7, wobei die Enden der Führungsstifte (42, 44) konisch sind.
